(19)

**Europäisches Patentamt**
**European Patent Office**
**Office européen des brevets**

(11) **EP 4 670 635 A1**

(12) **EUROPEAN PATENT APPLICATION**

(43) Date of publication:
**31.12.2025 Bulletin 2026/01**

(21) Application number: **24306007.6**

(22) Date of filing: **24.06.2024**

(51) International Patent Classification (IPC):
***A61B 6/00*** *(2024.01)*

(52) Cooperative Patent Classification (CPC):
**A61B 6/544; A61B 6/542; A61B 6/545;**
A61B 6/4441

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Ecential Robotics
38610 Gieres (FR)**

(72) Inventors:
- **LEVESY, Valentin
  38610 GIERES (FR)**
- **BERNIZET, François
  38610 GIERES (FR)**
- **GALLIX, Robin
  38610 GIERES (FR)**

(74) Representative: **Regimbeau
20, rue de Chazelles
75847 Paris Cedex 17 (FR)**

(54) **METHOD FOR CONTROLLING A SOURCE OF AN X-RAY IMAGING SYSTEM**

(57)    The invention relates to a method for controlling a source (101) of an X-ray imaging system (100), comprising:
- changing a desired output value of a tube current parameter of said source (100) from a first value to a second value different from the first value,
- computing a third value of the tube current parameter different from the second value such that the output tube current value becomes substantially equal to the second value within a predetermined time frame ($\Delta T$) from said change of the desired output value,
- providing said computed third value as a setpoint value for the source (101).

The invention also relates to a method for acquiring a plurality of 2D images of a region of interest of a patient's body (P) along a path of acquisition (T) with an X-ray imaging system (100), wherein acquisition parameters of the X-ray imaging system (100) vary along the path of acquisition (T), the acquisition parameters comprising a tube voltage parameter, a tube current parameter and an exposure time parameter, the method comprising:
a) determining a set of values of acquisition parameters for each acquired image along the path of acquisition (T);
b) if for an acquired image the value of the tube current parameter is modified with respect to a previous image acquisition, applying the above method to determine a third value to use as setpoint value for the source (101) of the X-ray imaging system (100), instead of the value determined in step a), to achieve the value determined in step a) as output of the source (101) at the time of the image acquisition.

FIGURE 3B

EP 4 670 635 A1

**Description**

**FIELD OF THE INVENTION**

The invention relates to a method for controlling a source of an X-ray imaging system. The invention also relates to a method of acquisition of a plurality of 2D images wherein acquisition parameters of the imaging system vary along the path of acquisition using the previous method to quickly vary the tube current output value of the source. Finally, the invention relates to a system to generate a 3D volume using the previous methods.

**BACKGROUND OF THE INVENTION**

X-ray imaging systems are frequently used during surgical procedures to provide physicians with image-based information about a patient's anatomical situation and/or the position and orientation of a surgical instrument with respect to the patient.

Such X-ray imaging systems typically provide two-dimensional (2D) projection images with different anatomical structures superimposed along the path of the X-rays.

A typical example of such a system for use in an intra-operative setting is the so-called C-arm which comprises a base frame on which a C-shaped arm is attached with several intermediate joints allowing moving the C-shaped arm in space along several degrees of freedom. One end of the C-shaped arm carries an X-ray source, and the other end carries an image detector.

Interventional procedures may be lengthy, and radiation dose therefore must be kept to a minimum to avoid radiation induced injury for both the patient and clinical staff. The image quality must be adequate to complete procedure while keeping the radiation dose as low as possible. The image needs to have signal to noise ratio at an acceptable level and sufficient contrast while respecting local standards or regulations and diagnostic reference levels for the patient dose.

The three major selectable parameters to determine the characteristics of the X-ray beam are the tube voltage (kV), tube current (mA), and exposure time (s). Often, the product of the tube current parameter and exposure time parameter is considered as one entity, the mAs (milliampere-second). Technically, mAs is a product of two units but, in common usage, its serves as quantity. More particularly, the tube voltage parameter is mostly responsible for the contrast of the acquired 2D X-ray image, while the product of the tube current parameter and exposure time parameter is mostly responsible for the signal to noise ratio of the acquired 2D X-ray images. When a plurality of 2D X-ray images need to be acquired, for example to reconstruct a 3D volume, the value of the exposure time parameter must stay low to avoid motion blur.

To determine the parameters to use for the X-ray imaging system for a specific patient and region of interest of the patient's body, a plurality of values is tested until values are found for each parameter to obtain X-ray images with an appropriate quality as previously mentioned.

The acquisition parameters of the X-ray imaging system can be determined only once, using a reference position of the imaging system, and used without any modifications for the acquisition of all the 2D images needed to reconstruct a 3D volume of the region of interest of the patient's body. Although simple and working fine most of the time, this approach has its drawbacks such as for patients having high body mass index (BMI) and/or osteoporosis.

Indeed, the patient's body does not have homogenous properties and those can vary greatly for the X-rays depending on the position of the C-arm, in relation to the region of interest, to acquire the 2D images. For example, to acquire a 2D image corresponding to a lateral view, the X-rays will need to cross more material (tissues, organs and/or bones) than in the case of a postero-anterior view. If the acquisition parameters are determined only once using the lateral view of the patient as reference to determine the acquisition parameters, the 2D image corresponding to the postero-anterior view may be at least partially burnt (i.e. with a noticeable number of saturated pixels with a white color) because there is less material to cross compared to the lateral view. Conversely, if the postero-anterior view is used as reference to determine the acquisition parameters, it may be harder to distinguish the different elements in the acquired 2D image corresponding to the lateral view since the X-rays will be more attenuated because there is more material to pass through compared to the postero-anterior view.

A solution would be to have acquisition parameters which vary along the path of acquisition of the imaging system to acquire the plurality of 2D images needed to reconstruct the 3D volume of a region of interest of the patient's body. However, while the change in value of the tube voltage parameter or of the exposure time parameter is instantaneous, the change in value of the tube current parameter is not instantaneous. Indeed, the filament of the X-ray source needs some time to heat and generate the required tube current value. This creates latency between each change in the value of the tube current parameter and may lead to the acquisition of 2D images with wrong associated tube current value since the filament may need more time than the time interval between the acquisition of two successive 2D images to reach the required value of the tube current parameter.

**BRIEF DESCRIPTION OF THE INVENTION**

A solution is wanted which works with any existing X-ray imaging, without modifying the structure of the X-ray source, which allows to quickly vary the value of the tube current parameters to acquire 2D X-ray images with the proper value of the intensity of the tube current without lengthening the time required for the acquisition of the plurality of 2D images.

A purpose of the invention is to propose a method addressing those issues.

According to a first aspect, the invention is directed towards a method for controlling a source of an X-ray imaging system, comprising:

- changing a desired output value of a tube current parameter of said source from a first value to a second value different from the first value,
- computing a third value of the tube current parameter different from the second value such that the output tube current value becomes substantially equal to the second value within a predetermined time frame from said change of the desired output value,
- providing said computed third value as a setpoint value for the source.

Some preferred but non-limiting features of the method described above are the following, taken alone or in any technically feasible combination:

- the third value is determined by:

  ○ modeling a response of the source of the X-ray imaging system to a change of value of the tube current parameter as a first order equation;
  ○ using the modelized response to determine the setpoint value to use in order to achieve, within the predetermined time frame, the desired output value;

- the predetermined time frame corresponds to a time interval between an acquisition of two successive 2D images by the X-ray imaging system;

According to a second aspect, the invention is directed towards a method for acquiring a plurality of 2D images of a region of interest of a patient's body along a path of acquisition with an X-ray imaging system, wherein acquisition parameters of the X-ray imaging system vary along the path of acquisition, the acquisition parameters comprising a tube voltage parameter, a tube current parameter and an exposure time parameter, the method comprising:

  a) determining a set of values of acquisition parameters for each acquired image along the path of acquisition;
  b) if for an acquired image the value of the tube current parameter is modified with respect to a previous image acquisition, applying the method describe above to determine a third value

to use as setpoint value for the source of the X-ray imaging system, instead of the value determined in step a), to achieve the value determined in step a) as output of the source at the time of the image acquisition.

Some preferred but non-limiting features of the method described above are the following, taken alone or in any technically feasible combination:

- step a) comprises the following steps:

  i) positioning the X-ray imaging system to at least two control positions and determining acquisition parameters for each of those control positions;
  ii) generating a model of the patient's body to obtain modelized properties of the patient's body;
  iii) using the modelized properties of the patient's body and the acquisition parameters of each control position to determine the set of values of acquisition parameters for each image acquisition of the X-ray imaging system along the path of acquisition;

- the at least two control positions correspond to a lateral view of the region of interest and a postero-anterior view of the region of interest;

- in step ii) the patient's body is modelized as an elliptical cylinder with uniform properties.

- the tube voltage parameter is fixed for each image acquisition;

- the method comprises before step iii):

  ○ defining a fixed tube voltage parameter;
  ○ replacing the tube voltage parameter of the acquisition parameters of each control position by the fixed tube voltage parameter; and
  ○ adjusting the tube current parameter and/or the exposure time parameter, of the acquisition parameters of each control position, to account for the modification of the tube voltage parameter;

- the fixed tube voltage parameter is determined by selecting the value of the tube voltage parameter of the acquisition parameters of one of the control positions;

- the fixed tube voltage is determined by computing an average value using the tube voltage parameter of each control position;

- the determination of acquisition parameter of step i) for one control position comprises:

  1) initializing the acquisition parameters with a set of values;

2) acquiring, with the X-ray imaging system at the control position, a 2D test image of the region of interest of the patient's body corresponding to the set of values;

3) computing a quality metric of the acquired 2D test image, comparing the quality metric of the acquired 2D test image with a target quality metric, and

- if the quality metric of the acquired 2D test image reaches the target quality metric, the set of values is used to define the acquisition parameters which will be used for the control position ;
- otherwise, the set of values is modified and step 2) to 3) are repeated, with the modified set of values, until the target quality metric is reached;

- if the tube current parameter is modified during step 3), the previous method is used to determine the third value to use as setpoint value of the source to acquire the 2D test image;

- only a sub-area of the 2D test image is used to compute the quality metric, the sub-area being centered on the region of interest of the patient's body.

According to a third aspect, the invention is directed towards a system to generate a 3D volume of a region of interest of a patient's body, said system comprising: an X-ray imaging system configured to acquire a plurality of 2D images and a control unit coupled to the X-ray imaging system, wherein the control unit is configured to:

- implement the above-described method to cause the X-ray imaging system to acquire the plurality of 2D images required to reconstruct a 3D volume;
- reconstruct the 3D volume using the plurality of 2D images.

**BRIEF DESCRIPTION OF THE DRAWINGS**

Further features and advantages of the invention will be apparent from the following description, based on the appended drawings wherein:

- FIG. 1 is a general overview of a surgical site including an operating table and a motorized C-arm;
- FIG. 2 schematically illustrates a motorized C-arm, an operating table and a patient's body;
- FIG. 3A illustrates the evolution of the output value of the tube current parameter of the source as a function of time when the desired output value is used as the input of the source.
- FIG. 3B illustrates the evolution of the output value of the tube current parameter of the source as a function of time when a transitory value, different from the desired output value, is used as the input of the source.
- FIG. 4 schematically illustrates a path of acquisition of the imaging system with the position of two control position according to the invention.

For sake of legibility of the drawings, the figures are not necessarily drawn to scale. The reference signs identical from one figure to another one designate the same elements or elements fulfilling the same function.

**DETAILED DESCRIPTION OF EMBODIMENTS**

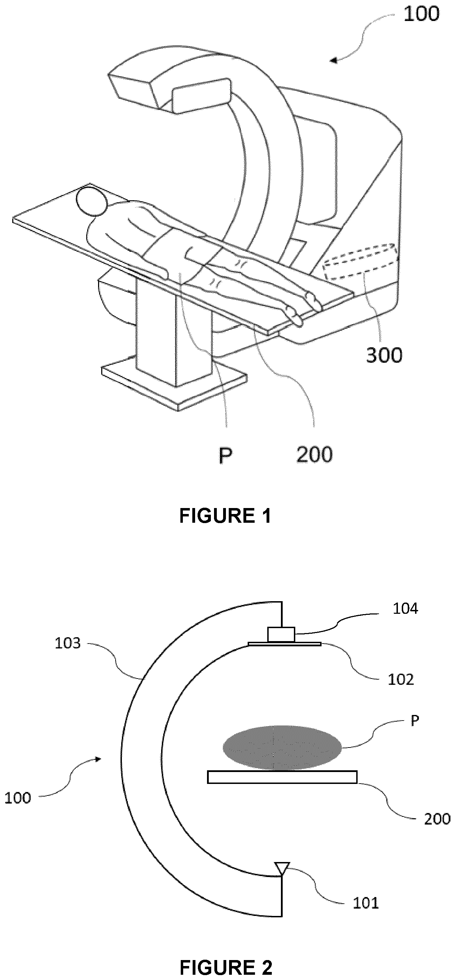

FIG.1 is a general overview of a surgical site including an operating table 200, an X-ray imaging system 100 which is, in this case, a motorized C-arm, and a patient P lying on the operation table 200.

X-ray imaging system

As depicted in more details in FIG.2, the X-ray imaging system 100 comprises at least one X-ray source 101 and at least one X-ray image detector 102. The X-ray imaging system 100 produces at least one 2D X-ray image that is the result of a conical projection of a patient's anatomy, wherein the tip of the cone is approximately the central point of the X-ray source 101 and the basis of the cone is approximately the portion of the X-ray image detector 102 that is reached by X-ray beams that have been collimated in a given shape and orientation.

For example, the X-ray imaging system 100 can be a conventional C-arm, or any Cone-Beam Computed Tomography (CBCT), such as the Surgivisio device (Surgivisio, Gieres, France), or Vision FD Vario 3D (Ziehm), CIOS Spin Mobile 3D (Siemens), Airo (Stryker), Loop-X (Brainlab), O-arm (Medtronic).

A conventional C-arm is designed to allow the X-ray source 101 and X-ray detector 102 to rotate along a C-shaped gantry while obtaining projection images of the patient P placed between the X-ray source 101 and the X-ray detector 102 of the gantry.

A CBCT has a mobile X-ray source 101 and a mobile X-ray image detector 102, wherein the X-ray source and the X-ray image detector have motorized motions, moving together or independently. A CBCT can have a C-arm shape or an O-arm shape. It can be used to acquire a set of 2D X-ray images over approximately 180° of orbital rotation that can be combined with translations and from which a 3D volume can be reconstructed using tomography algorithms or tomosynthesis algorithms.

The X-ray imaging system 100 may be motorized, notably the C-shaped arm may comprise motors allowing movement horizontally, vertically and around the swivel axes, so that 2D X-ray images of the patient P are produced from almost any angle. Each motor is associated to an encoder that provides at any time the relative position of the medical imaging system 100 with respect to a reference position. When a 2D X-ray image is acquired, the corresponding position of the imaging system 100 is recorded. Thus, each 2D image is recorded in the coordinate system of the imaging system.

The X-ray source 101 generates the X-rays which are produced when highly energetic electrons interact with matter, converting some of their kinetic energy into electromagnetic radiation.

The X-ray source 101 comprises:

- an X-ray tube to generate the X-rays, the X-ray tube containing an electron source, such as a filament made of tungsten wire, and a target electrode, on which the electrons impact, within an evacuated glass or metal envelope;
- a tube housing to provide protective radiation shielding and cool the X-ray tube;
- an X-ray generator to supply the voltage to accelerate the electrons and control the X-ray beams characteristics through the selection of the tube voltage, tube current and exposure time; and
- a collimator to define the size and shape of the X-ray field incident on the patient.

The X-ray generator supplies the electrical power and permits selection of the value of the tube voltage, tube current, and exposure time parameters. Depending upon the type of imaging examination and the characteristics of the anatomy being imaged, the tube voltage parameter, measured in kilovolt (kV), is generally set to values from 40 to 150 kV for diagnostic imaging and 25 to 45 kV for mammography. The tube current parameter, measured in milliamperes (mA), is proportional to the number of electrons per second flowing from the cathode to the anode. For projection radiography, the tube current parameter is generally set from 50 to 120 mA in conjunction with short exposure times (typically less than 100 ms).

As shown in FIG. 2, the X-ray imaging system generally comprises an anti-collision device 104 fixed to a movable part of the C-arm. Said anti-collision device may be activated to detect obstacles in the vicinity of said movable part of the C-arm when it is moving. Such an anti-collision device may typically be used in a preliminary path of the C-arm, before launching acquisition of a set of 2D X-ray images, in order to make sure that said path does not involve any collision of the C-arm with the patient or any other object in the environment of the patient, such as the operating table. The anti-collision device may also be activated during acquisition of a set of 2D X-ray images, in order to prevent any collision of the C-arm with the patient or any other object in the environment of the patient, in particular if the environment has changed since the preliminary path.

The anti-collision device may rely on various sensor technologies to detect an obstacle in the vicinity of the C-arm. Said obstacle may be the patient's body or any object located in the vicinity of the patient. For example, but non-limitatively, the anti-collision device may comprise a proximity sensor (e.g. a capacitive sensor), a telemeter, a LIDAR (Laser Imaging, Detection And Ranging), a stereo camera and/or a tactile sensor.

For sake of simplicity, the terms "2D X-ray image", "X-ray imaging system" and "X-ray source", will be referred to as "2D image", "imaging system" and "source" respectively in the following description.

Control unit

In the embodiment of FIG.1, the control unit 300 is embedded in the imaging system 100.

In other embodiments (not illustrated), the control unit 300 may be provided separate from the imaging system 100 and may be configured to communicate wirelessly or by wires with the imaging system 100. For example, the control unit 300 may be embedded in a separate station comprising a user interface and including batteries.

The control unit 300 comprises at least one processor configured to implement algorithms designed to carry out the method of the invention that will be described in more details in the following description. The control unit 300 also comprises one communication device and at least one data storage device to store the algorithms and various information such as the allowed ranged for each parameter, table of values etc. The communication device is used to communicate with the imaging system 100, more particularly with the source 101 of the imaging system 100, to implement the method of the invention.

The anti-collision device is coupled to the control unit, so as to send to the control unit data regarding potential obstacles, such as a distance between the anti-collision device and an object. The control unit may be configured to stop the movement of the C-arm in case the anti-collision device has detected an object at a distance smaller than a predetermined distance from the moving part of the C-arm.

General presentation of the method

As mentioned in the introduction, after a change of the desired output value of the tube current parameter, the source 101 does not achieve instantaneously an output tube current value substantially equal to the changed desired output value. A delay occurs, generally longer than the time interval between the acquisition of two successive 2D images by the imaging system 100, to achieve the changed desired output value. This leads to incompatibility for application, such as the acquisition of a plurality of 2D images to generate a 3D volume, where the output value of the tube current parameter must achieve the changed desired output value no later than the time of acquisition of the next 2D image.

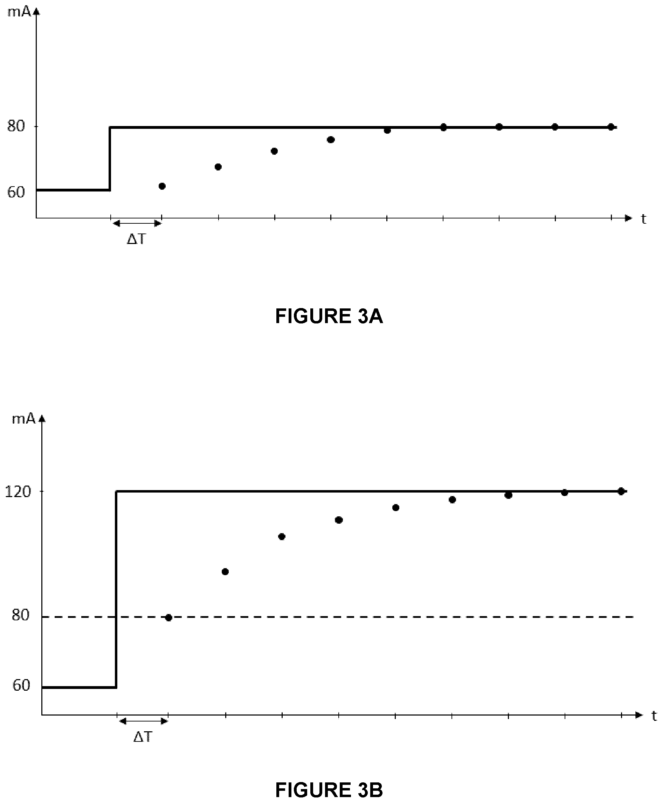

Indeed, the response of the source 101 to a change of the desired value of the tube current parameter (considered as a pulse) is not a square wave but is similar to a first order response. For example, if the desired output value of the tube current parameter is 60 mA and the new desired output value is 80 mA, a delay equal to the time interval needed to acquire approximately five 2D images occurs as illustrated in FIG.3A.

To avoid this issue, it may be preferred to only modify the exposure time parameter to vary the value of the product of the tube current parameter with the exposure time parameter. Indeed, as mentioned in the introduction, this product is responsible for the signal to noise ratio of the acquired 2D image. So, as long as the value of this product is not modified, it is possible to adjust value of the tube current parameter and/or of the exposure time parameter. However, it is not always possible to only modify the exposure time parameter because of the limitation of the range of values possible for the exposure time parameter, in particular to avoid blur effect when acquiring a plurality of 2D images along a path of acquisition. There are always cases where it is necessary to change the desired output value of the tube current parameter, resulting in a latency problem.

To address this latency problem, the invention provides a method to be implemented when the desired output value of the tube current parameter is changed from a first value to a second value different from the first value, wherein a third value of the tube current parameter different from the second value is computed such that the output tube current value becomes substantially equal to the second value within a predetermined time frame from said change of the desired output value, and said computed third value is provided to the source as a setpoint value for the source.

For example, if the output parameter changes from a first value to a second value greater than the first value, the third value, which is the setpoint value of the source, is greater than the second value. Conversely, if the second value is smaller than the first value, the third value is smaller than the second value. Otherwise said, the third value evolves from the first value in the same direction as the second value, but presents a greater difference with the first value, so as to impose a quicker adjustment of the output of the source to reach the second value.

This method is implemented by the control unit.

Preferentially, the predetermined time frame ΔT corresponds to the time interval between the acquisition of two successive 2D images by the imaging system to acquire the next 2D image with the desired output value for the tube current parameter.

The third value, which can be considered as a transitory tube current parameter, is used to compensate for latency, and is determined by:

- modeling the response of the source 101 of the imaging system 100 to a change of value of the tube current parameter as a first order equation;

- using the modelized response to determine the transitory tube current parameter to use in order to achieve, within the predetermined time frame ΔT, the changed desired output value.

The modeling of the response of the source 101 is recomputed for each modification of the desired output value of the tube current parameter.

The response of the source 101 of the imaging system 100 is modelized using the following equation:

$$mA(t) = A\left[1 - e^{-\frac{t}{B}}\right]$$

where:

- $mA(t)$ represents the time response of the tube current parameter to a change of the setpoint value of the tube current value parameter;
- $t$ is the time parameter;
- A represents a constant linked to the setpoint value;
- B represents the system time constant.

To obtain the value of A and B, the evolution of the value of the output of the source is measured over time, when the setpoint value of the source is changed from a previous value C to a new value A'. The value C can be equal to 0 and the value A' must be higher than C. The values A and C can be selected arbitrary for the purpose of determining the value of A and B.

The parameters A and B are obtained as follows:

- A is obtained by computing A = A' - C
- B is obtained by finding B such as $mA(B) = (1 - e^{-1}) * A + C$.

This modelized response is then used to determine the transitory tube current parameter to use as setpoint A' to achieve, within the predetermined time frame ΔT, an output tube current value $mA(\Delta T)$ substantially equal to the changed desired output value. "Substantially equal" means that the value obtained as output does not differ from the changed desired output value by more than 10% and advantageously by more than 5%.

The source power supply is stopped or changed once the output reaches the desired output value and the 2D image is acquired. The output value of the source then never reaches the value of the transitory tube current parameter used as input. It is then possible to use, as value of the transitory tube current parameter, a value exceeding the maximal allowed value for the tube current parameter (i.e., in relation with regulation or local standards).

The 2D image acquired then properly corresponds to the desired output value of the tube current parameter.

FIG.3A and FIG.3B illustrate the evolution of the output value of the tube current parameter (mA) of the source as a function of time (t) when using two different scenarios for the value of the setpoint value, as described below, when the desired output value changes from 60mA to 80mA. The bold line represents the desired output value, and the spots represent the output value of the source at each predetermined time frame $\Delta T$. In this example, the predetermined time frame $\Delta T$ corresponds to the time interval between the acquisition of two successive 2D images.

FIG.3A illustrates the situation where the desired output value of the tube current parameter is used as setpoint.

FIG.3B illustrates the situation where a transitory value of 120mA is used as setpoint instead of the desired output value.

It can be seen that the value of the desired output is reached far quicker, in a single predetermined time frame $\Delta T$, when applying the method of the invention (FIG.3B) compared to the situation of FIG.3A. In the case of FIG.3A, to achieve the desired output value, a waiting time of five time the time interval $\Delta T$ will be required, considerably lengthening the time to acquire the needed 2D images. In addition, when acquiring a plurality of 2D images, it may be necessary to use a variable motion speed of the imaging system along the path of acquisition since, for some 2D images, no modification of the output value of the tube current parameter will be needed.

Application of the method for the acquisition of 2D images to reconstruct a 3D volume

As mentioned in the introduction, since the patient's body does not have homogenous properties, it is advantageous to acquire the plurality of 2D images required for the 3D volume with acquisition parameters which vary along the path of acquisition T of the imaging system 100.

To achieve this goal, the method of the invention comprises the following steps, that are implemented by the control unit:

> a) determining the set of values of acquisition parameters for each image acquisition of the imaging system 100 along the path of acquisition T;
> b) if for an image acquisition the value of the tube parameter is modified with respect to a previous image acquisition, apply the method previously described to determine the transitory tube current parameter to use as setpoint value for the source 101 of the imaging system 100, instead of the value determined in step a), to achieve the value determined in step a) as output of the source 101 at the time of the image acquisition.

To determine the set of values of acquisition parameters for each image acquisition, step a) comprises the following steps:

> i) positioning the imaging system 100 to at least two control positions C1, C2 and determining acquisition parameters for each of those control positions C1, C2
> ii) modeling the patient's body P to obtain modelized properties of the patient's body P;
> iii) using the modelized properties of the patient's body P and the acquisition parameters of each control position C1, C2 to determine the set of values of acquisition parameters for each image acquisition of the imaging system 100 along the path of acquisition T.

The term "control position" refers to the position of either the source 101 or the image detector 102 of the imaging system 100. The control positions C1, C2 need to be spaced far enough apart to give useful information about the non-uniformity of the body's properties.

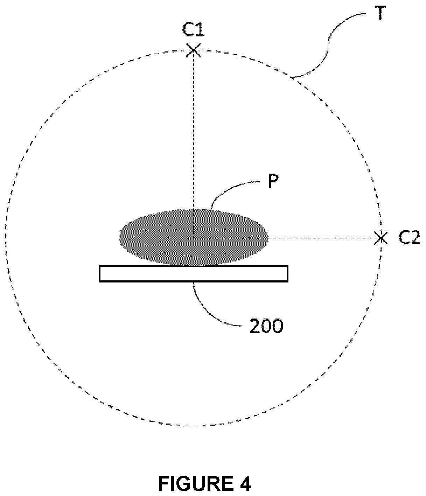

FIG.4 schematically illustrates the path of acquisition T of the imaging system with the position of two control positions C1, C2. In this figure, the path of acquisition T has a circular trajectory around the patient but a more complex trajectory could be used to generate the 3D volume.

Advantageously, as illustrated in FIG.4, the at least two control positions C1, C2 correspond to a lateral view C2 (where there is the biggest amount of material to pass through) and a postero-anterior view C1 (where there is the less amount of material to pass through) of the region of interest. Those two control positions allow to set the minimal and maximal values of the acquisition parameters along the path of acquisition.

More than two control positions could be used. The greater the number of control positions, the better the extrapolation of the acquisition parameters between those positions, but the higher the patient's exposure to radiation.

In a preferred embodiment, in step ii) the patient's body P is modelized as an elliptical cylinder with uniform properties. The dimensions of the patient's body are used to define the shape of the elliptical cylinder.

Said dimensions may be estimated in different ways.

Preferably, the anti-collision system of the X-ray imaging system may be used not only to detect obstacles and prevent a collision with such obstacles, as provided by the conventional use of such an anti-collision device, but also (or alternatively) to detect an outer surface of the patient's body and record the position of each detected point of this outer surface along the path of the C-arm. It is to be noted that this detection does not require changing the design of the anti-collision device, since the detection of an obstacle is based on measurement of a distance between the anti-collision device and the obstacle (the outer surface of the obstacle being detected by the sensor(s) of the anti-collision device). In particular, all anti-collision devices mentioned above allow detecting the outer surface of the patient's body. However, in conventional use of the anti-collision device, no recording is made of the distance between the anti-collision device and the obstacle along the path of the C-arm. During a path of the C-arm, the anti-collision device may thus send volumetric data of the patient's body to the control unit. Said data may be raw sensor data, for example in the form of an electric signal, a video signal or any other type of signal depending on the anti-collision technology, issued by the anti-collision device. The control unit then treats the raw sensor data to compute volumetric data of the patient's body. Alternatively, the anti-collision device may itself send volumetric data to the control unit. In practice, it is generally not necessary to obtain volumetric data of the whole patient's body, but only of a portion of the patient's body enclosing a region of interest which is to be imaged by the C-arm. For example, if the region of interest is a vertebra, the portion of the patient's body may be a portion of the patient's torso enclosing said vertebra and, if appropriate, adjacent vertebrae. The volumetric data received or computed by the control unit may be typically a set of distances between the patient's body and the anti-collision device along the path of the motorized C-arm. For more detail about the construction of a model of the patient's body using the anti-collision device, one may refer to document WO2023/275087.

Alternatively, the dimensions of the patient's body may be measured by the clinical staff pre- or intra-operatively, for example using a measuring tape, a ruler or any other suitable size measurement device.

In other embodiments, dimensions of the patient's body may be included in the surgical plan which is generated by the surgeon and communicated to the control unit, and retrieved from the surgical plan to compute a model of the patient's body.

In step iii), the elliptical cylinder is used to estimate the distance crossed by the X-rays through the patient's body to determine the percentage attenuation by comparison with the distance associated to the two control positions C1, C2 which respectively correspond to the minimal and maximal distance. This percentage attenuation is then used to deduce the evolution of the set of value of the acquisition parameters along the path of acquisition T for each image acquisition.

Optionally, other more complex methods can be used to model the patient's body P in step ii) and/or to determine the set of values of acquisition parameters of step iii).

Advantageously, the tube voltage parameter is fixed during the path of acquisition T of the imaging system 100. Indeed, since the tube voltage parameter is linked to the contrast of the acquired 2D image, a change of value of the tube voltage parameter during the acquisition of the 2D images will lead to the acquisition of 2D images with various contrast properties. The 3D volume reconstructed from such 2D images will not have ideal properties and may contain visual artifact which may lead to erroneous interpretations. To generate a 3D volume with appropriate properties, the method comprises before step iii):

- defining a fixed tube voltage parameter;
- replacing the tube voltage parameter of the acquisition parameters of each control position C1, C2 by the fixed tube voltage parameter; and
- adjusting the tube current parameter and/or the exposure time parameter, of the acquisition parameters of each control position, to account for the modification of the tube voltage parameter.

In a first embodiment, the fixed tube voltage parameter is determined by selecting the value of the tube voltage parameter of the acquisition parameters of one of the control positions C1, C2.

In a second embodiment, the fixed tube voltage is determined by computing an average value using the tube voltage parameter of each control position C1, C2.

To determine the acquisition parameters of each control position C1, C2 of step i), different sets of values are tested until an acceptable 2D test image is acquired for each control position. The set of values associated to this 2D test image defines the acquisition parameters used for the associated control position. The method comprises, for each control position:

1) initializing the acquisition parameters with a set of values;
2) acquiring, with the imaging system 100 at the control position C1, C2, a 2D test image of the region of interest of the patient's body P corresponding to the set of values;
3) computing a quality metric of the acquired 2D test image, comparing the quality metric of the acquired 2D test image with a target quality metric, and

• if the quality metric of the acquired 2D test image reaches the target quality metric, the set of values is used to define the acquisition parameters which will be used for the control position C1, C2;
• otherwise, the set of values is modified and step b) to c) are repeated, with the modified set of values, until the target quality metric is reached.

In step 1) the acquisition parameters of the imaging system 100 are initialized with a set of values. The set of values comprises a value for each parameter (i.e. tube voltage parameter, tube current parameter and exposure time parameter).

Advantageously, the set of values comprises only two values: a value for the tube voltage parameter and a value for the product of the tube current parameter with the exposure time parameter, as this product is often considered as one entity as mentioned before.

The set of value used to initialize the acquisition parameters of step 1) can be chosen arbitrarily, such as using the first set of values of a predefined table, or theoretically estimated using patient information, for example with a method estimating the equivalent water thickness of the patient's body. This set of values could also be selected among a list of predetermined set of values by selecting the most similar set of values to the one theoretically estimated.

In step 2), once the set of values to initialize the acquisition parameters is defined, a 2D test image of the region of interest of the patient's body is acquired with the imaging system 100.

In step 3), a quality metric of the acquired 2D test image is computed and compared with a target quality metric to evaluate the set of values. The quality metric is selected among metric used to qualify the quality of an image, such as metric based on contrast, edge or brightness of an image. The target quality metric corresponds to either the quality metric of a reference image or depends on properties of the image detector 102 of the imaging system 100. For example, the quality metric may be based on the brightness of the image and comprises computing the average pixel intensity value of the image.

Advantageously, only a sub-area of the 2D test image is used to compute the quality metric, the sub-area being centered on the region of interest of the patient's body P. Indeed, the most critical part of the 2D test image, and of the 3D volume, is the area near the region of interest, whereas it is less important if the surrounding area is blurred, saturated with white or black pixel, or has a low contrast. This is particularly impacting in the case where the patient's body is equipped with a registration phantom comprising radio-opaque fiducials which need to be included in the 3D volume. The registration phantom being disposed on top of the patient's body, an important air volume will be included in the 3D volume in addition to the radio-opaque fiducials. The air volume is generally associated to a huge black area on the 2D while the radio-opaque fiducials generate very bright small areas. The use of a sub-area centered on the region of interest of the patient's body allow to ignore the pixel associated to the air volume and to the radio-opaque fiducials, which generally correspond to the extremal pixel intensity values of the image, to select a tube voltage parameter which led to the best contrast in this sub-area. It is not important if the choice of the tube voltage parameter leads to saturated pixels for the radio-opaque fiducials outside of the sub-volume since it is only necessary to be able to estimate the position of their center for registration purposes and/or for generating a 3D volume.

If the quality metric of the acquired 2D test image reaches the target quality metric, the current set of values of the acquisition parameters is used to define the acquisition parameters which will be used for subsequent acquisition of 2D X-ray images of the region of interest of the patient's body. By "reaching the target quality metric", it must be understood that the quality metric of the 2D test image does not differ, from the target quality, by more than 10%.

Otherwise, the set of values is modified and step 2) to 3) are repeated, with the new modified set of values, until the target quality metric is reached. The set of values may be tune lower or higher depending on the value of the quality metric of the 2D test image compared to the target quality metric. For example, when using a quality metric based on the brightness of the image, the set of values is tune lower when the quality metric of the image is higher than the target quality metric and tune higher otherwise.

If during step 3), the value of the tube current parameter is modified, the previously described method is used to determine the transitory tube current parameter to use as setpoint to acquire the 2D test image. As mentioned before, it is not always possible to only modify the value of the exposure time parameter to vary the value of the product of the tube current parameter with the exposure time parameter.

Once the set of values of the acquisition parameters to use for a specific control position is obtained, it is still possible to adjust the individual value of the tube current parameter and the exposure time parameter as long as the value of their product is not modified. This is particularly interesting, if to avoid the latency issue for the modification of the tube current parameter, the value of the exposure time parameter is higher than the maximal value of the exposure time not leading to blur effect when acquiring a plurality of 2D image along a path of acquisition.

This application is advantageous because it allows to generate a 3D volume with a better quality than the 3D volume obtained with fixed acquisition parameters along the path of acquisition without lengthening the time required to acquire all the 2D image.

This application is also advantageous because it reduces the irradiation exposure of the patient since there is no need to compute, and acquire 2D test images, for each position of the imaging system 100 along the path of acquisition. The method only needs to be applied to at least two selected control positions, and acquisition parameters between those positions will be extrapolated using the modelized properties of the patient's body.

Additionally, with this application, all the acquisition parameters are determined before starting the acquisition of the plurality of 2D image so there is no need to pause the acquisition of the 2D image to compute, at each image acquisition, the value of the acquisition parameter.

**Claims**

1. Method for controlling a source (101) of an X-ray imaging system (100), comprising:

    - changing a desired output value of a tube current parameter of said source (100) from a first value to a second value different from the first value,

- computing a third value of the tube current parameter different from the second value such that the output tube current value becomes substantially equal to the second value within a predetermined time frame ($\Delta T$) from said change of the desired output value,

- providing said computed third value as a setpoint value for the source (101).

2. Method according to claim 1, wherein the third value is determined by:

- modeling a response of the source (101) of the X-ray imaging system (100) to a change of value of the tube current parameter as a first order equation;

- using the modelized response to determine the setpoint value to use in order to achieve, within the predetermined time frame ($\Delta T$), the desired output value.

3. Method according to any of claim 1 to 2, wherein the predetermined time frame ($\Delta T$) corresponds to a time interval between an acquisition of two successive 2D images by the X-ray imaging system (100).

4. Method fof acquiring a plurality of 2D images of a region of interest of a patient's body (P) along a path of acquisition (T) with an X-ray imaging system (100), wherein acquisition parameters of the X-ray imaging system (100) vary along the path of acquisition (T), the acquisition parameters comprising a tube voltage parameter, a tube current parameter and an exposure time parameter, the method comprising:

a) determining a set of values of acquisition parameters for each acquired image along the path of acquisition (T);

b) if for an acquired image the value of the tube current parameter is modified with respect to a previously acquired image, applying the method of any of claims 1 to 3 to determine a third value to use as setpoint value for the source (101) of the X-ray imaging system (100), instead of the value determined in step a), to achieve the value determined in step a) as output of the source (101) at the time of acquisition of the respective image.

5. Method according to claim 4 wherein step a) comprises:

i) positioning the X-ray imaging system (100) to at least two control positions (C1, C2) and determining acquisition parameters for each of said control positions (C1, C2);

ii) generating a model of the patient's body (P) to obtain modelized properties of the patient's body (P);

iii) using the modelized properties of the patient's body (P) and the acquisition parameters of each control position (C1,C2) to determine the set of values of acquisition parameters for each image acquisition of the X-ray imaging system (100) along the path of acquisition (T).

6. Method according to claim 5, wherein the at least two control positions (C1, C2) correspond to a lateral view of the region of interest and a postero-anterior view of the region of interest.

7. Method according to any of claim 5 to 6, wherein in step ii) the patient's body (P) is modelized as an elliptical cylinder with uniform properties.

8. Method according to any of claim 5 to 7, wherein the tube voltage parameter is fixed for each image acquisition.

9. Method according to claim 8 and any of claim 5 to 7, wherein the method comprises before step iii):

- defining a fixed tube voltage parameter;

- replacing the tube voltage parameter of the acquisition parameters of each control position (C1,C2) by the fixed tube voltage parameter; and

- adjusting the tube current parameter and/or the exposure time parameter, of the acquisition parameters of each control position, to account for the modification of the tube voltage parameter.

10. Method according to claim 9, wherein the fixed tube voltage parameter is determined by selecting the value of the tube voltage parameter of the acquisition parameters of one of the control positions (C1, C2).

11. Method according to claim 9, wherein the fixed tube voltage is determined by computing an average value using the tube voltage parameter of each control position (C1, C2).

12. Method according to any of claim 5 to 11, wherein the determination of acquisition parameter of step i) for one control position (C1, C2) comprises:

1) initializing the acquisition parameters with a set of values;

2) acquiring, with the X-ray imaging system (100) at the control position (C1, C2), a 2D test image of the region of interest of the patient's body (P) corresponding to the set of values;

3) computing a quality metric of the acquired 2D test image, comparing the quality metric of the acquired 2D test image with a target quality metric, and

*if the quality metric of the acquired 2D test image reaches the target quality metric, the set of values is used to define the acquisition parameters which will be used for the control position (C1, C2);

*otherwise, the set of values is modified and step 2) to 3) are repeated, with the modified set of values, until the target quality metric is reached.

13. Method according to claim 12, wherein if the tube current parameter is modified during step 3), the method of any of claim 1 to 3 is used to determine the third value to use as setpoint value of the source to acquire the 2D test image.

14. Method according to any of claim 12 to 13, wherein only a sub-area of the 2D test image is used to compute the quality metric, the sub-area being centered on the region of interest of the patient's body (P).

15. System to generate a 3D volume of a region of interest of a patient's body (P), said system comprising an X-ray imaging system (100) configured to acquire a plurality of 2D images and a control unit (300) coupled to the X-ray imaging system (100), wherein the control unit (300) is configured to:

- implement the method of any of claim 4 to 14 to cause the X-ray imaging system to acquire the plurality of 2D images required to reconstruct a 3D volume;
- reconstruct the 3D volume using the plurality of 2D images.

**FIGURE 1**

**FIGURE 2**

**FIGURE 3A**

**FIGURE 3B**

**FIGURE 4**

Europäisches
Patentamt
European
Patent Office
Office européen
des brevets

# EUROPEAN SEARCH REPORT

**Application Number**

EP 24 30 6007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/126216 A1 (LIU JAMES Z [US] ET AL) 27 April 2023 (2023-04-27) | 1-4,15 | INV.<br>A61B6/00 |
| Y | * paragraph [0019] - paragraph [0057]; figures * | 5-14 | |
| Y | JP 2006 116137 A (HITACHI MEDICAL CORP) 11 May 2006 (2006-05-11) * paragraph [0028] - paragraph [0085]; figures * | 5-14 | |
| Y | US 2005/185759 A1 (TOTH THOMAS L [US] ET AL) 25 August 2005 (2005-08-25) * paragraph [0034] - paragraph [0048]; figures * | 5-7 | |

**TECHNICAL FIELDS
SEARCHED (IPC)**

A61B
H05G

The present search report has been drawn up for all claims

| Place of search | Date of completion of the search | Examiner |
|---|---|---|
| Munich | 23 October 2024 | Strubel, Christine |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
 
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 30 6007

This annex lists the patent family members relating to the patent documents cited in the above-mentioned European search report.
The members are as contained in the European Patent Office EDP file on
The European Patent Office is in no way liable for these particulars which are merely given for the purpose of information.

23-10-2024

| Patent document cited in search report | | Publication date | Patent family member(s) | | Publication date |
|---|---|---|---|---|---|
| US 2023126216 | A1 | 27-04-2023 | CN | 115998317 A | 25-04-2023 |
| | | | US | 2023126216 A1 | 27-04-2023 |
| JP 2006116137 | A | 11-05-2006 | JP | 4731151 B2 | 20-07-2011 |
| | | | JP | 2006116137 A | 11-05-2006 |
| US 2005185759 | A1 | 25-08-2005 | CN | 1657008 A | 24-08-2005 |
| | | | DE | 102005007190 A1 | 15-09-2005 |
| | | | JP | 4746890 B2 | 10-08-2011 |
| | | | JP | 2005230547 A | 02-09-2005 |
| | | | NL | 1028294 C2 | 29-11-2006 |
| | | | US | 2005185759 A1 | 25-08-2005 |

EPO FORM P0459

For more details about this annex : see Official Journal of the European Patent Office, No. 12/82

**EP 4 670 635 A1**

**Patent documents cited in the description**

- WO 2023275087 A